(19) Europäisches Patentamt
European Patent Office
Office européen des brevets

(11) Publication number: **0 385 401 A1**

(12) **EUROPEAN PATENT APPLICATION**

(21) Application number: 90103808.3

(22) Date of filing: 27.02.90

(51) Int. Cl.5: C12N 15/55, C12N 1/20, C12N 9/20, C12N 9/16, C11D 3/386, D06L 1/12, //C12N1:20,C12R1:38

(30) Priority: 27.02.89 US 316043

(43) Date of publication of application: 05.09.90 Bulletin 90/36

(84) Designated Contracting States: BE DE FR GB NL

(71) Applicant: OCCIDENTAL CHEMICAL CORPORATION
P.O. Box 189
Niagara Falls New York 14302(US)

(72) Inventor: Pierce, George E.
70 A Cokesbury Road, Lebanon
New Jersey 08833(US)
Inventor: Wick, Carolyn B.
8722 Olentangy River Road
Delaware, Ohio 43015(US)
Inventor: Palmer, Donna T.
24 East Park Street
Westerville, Ohio 43081(US)

(74) Representative: Vossius & Partner
Siebertstrasse 4 P.O. Box 86 07 67
D-8000 München 86(DE)

# (54) Unique Microbial lipases with activity at temperatures and pHs suitable for use in detergents.

(57) Disclosed is a biologically pure culture of a bacterium having ATCC number 53848, 53849, 53850, 53851, 53852, 53853, a genetic equivalent thereof, or a mixture thereof. Also disclosed are substantially pure lipases which have maximal lipolytic activity between 20 °C and 85 °C at a pH between 8.0 and 11, where the lipase is produced from genetically active material substantially identical to the lipase-encoding genetic material of a microorganism capable of surviving and propagating in an environment of 100 ppm of 3,4-dichlorobenzoate. Also disclosed is a method of preparing the lipases from microorganisms, such as the claimed bacteria, lipase compositions, detergents, and a method of using the detergents.

EP 0 385 401 A1

# UNIQUE MICROBIAL LIPASES WITH ACTIVITY AT TEMPERATURES AND PHS SUITABLE FOR USE IN DETERGENTS

The present invention relates to unique lipases, and more particularly to lipases adopted for use in detergent compositions.

While a good modern detergent must be capable of removing many different types of soil from fabrics, this invention is primarily directed at the problem of removing fatty and oily type stains. Traditional synthetic detergents contain high levels of sodium tripolyphosphate (STPP) and related compounds as builders, which raise the pH of the wash water and aid in the emulsification of fats and oils, thereby effecting their removal from fabrics. However, the removal or reduction of phosphates from detergent compositions in recent years, largely because of environmental concerns, led to the need for detergent producers to incorporate other cleaning compounds, such as enzymes, into detergent compositions.

The use of enzymes in detergents has been known in the art for many years. In German Patent 283,923 (1915) Rohm demonstrated that fabrics could be cleaned more effectively and at lower temperatures when pretreated with pancreatic enzymes that hydrolyze proteins and fats. However, these enzymes were derived from animal pancreases and were not practical for use in detergents. Enzymes that degrade proteins (i.e., proteases), and enzymes that degrade starches (i.e., amylases), were first widely used in industrial and household laundry detergent and laundry presoak/prespot compositions in the 1960's. Today, commercial protease and amylase detergent additives which are active and stable at the temperatures and pHs normally associated with current washing solutions are readily available and are widely used in commercial detergents in Europe, the United States, and Japan.

However, while traditional synthetic detergents employ very high temperatures and alkalinities for maximum effectiveness, the newer protease and amylase-based detergents require lower temperatures and lower alkalinities for maximum effectiveness. Furthermore, the combined effects of less STPP in modern detergent compositions and the lower temperatures and lower pHs in current wash water solutions significantly reduce the fat and oil cleaning power of such detergents. Therefore, there is a need for a "fatty-stain" hydrolyzing enzyme (i.e., a lipase) which is suitable for use in detergents.

Most of the research into the development and application of lipases has been associated with food processing, and lipases used in foods do not function well in the pH and temperature conditions of wash water detergent solutions. Furthermore, food-grade lipases have been found to be generally incompatible with other ingredients normally found in detergent compositions, especially anionic surfactants (see European Patent Application 0,097,810), which reduce their activity, and proteases, which may attack and degrade the lipase, which is itself a protein.

There has been considerable experimental work on the use of lipases as detergent additives. (See Andree et al., J. Appl. Biochem 2 (1980) 218-229, for a review of lipases as detergent components.) In recent years several lipases have been developed which are intended for use in laundry detergent compositions. Examples include "Amano P" (see Japanese patent 63039579) and Lipolase(TM) 30-T (see European Patent Application 258068). However, these lipases have their maximal activity at temperatures and pHs which are too low for laundry detergent solutions. They therefore appear to be better suited to presoaking/prespotting applications, where the pH is about 7 to 8 and the temperature is less than 40° C, than to laundry detergent solutions, where the pH is typically in the range of 8.5 to 11.0 and the temperature up to 80° C. A lipase suitable for use in detergents should not only have its maximal activity within the temperature and pH ranges of washing solutions, but also should demonstrate stable activity in the presence of other components in the detergent, such as proteases and surfactants.

Accordingly, it is a primary objective of the present invention to provide lipases which are active within the temperature and pH ranges of synthetic detergents, and compatible with synthetic detergent compositions.

Another objective of the present invention is to provide novel lipases produced extracellularly from microorganisms obtained from unique environments.

Yet another objective of the present invention is to provide stable and cleaning-effective lipase compositions which can be used in a variety of processes and applications such as in cleaners and degreasers, and in biotransformation applications in chemical, pharmaceutical, food, cleaning, and waste treatment processes.

Still yet another objective of the present invention is to provide a process of selecting, isolating, culturing, and propagating certain pure cultures of particular naturally occurring microorganisms that are capable of producing lipases uniquely suitable for use in detergents and other cleaning, degreasing, and biotransformation applications.

These and other objectives, as well as the method and manner of accomplishing them, will become apparent from the detailed description of the invention which follows.

We have found that certain novel and unique lipases can be produced from pure cultures of microorganisms that live in environments containing a high concentration of halogenated organic compounds. There are negliglible levels of fats and ester-linked oils which could serve as microbial nutrients in these environments. Thus, it is surprising and unexpected that organisms in these environments would produce these unique lipases. To someone skilled in the art, there is no apparent reason to expect these microorganisms to produce these unique lipases.

Even more surprising, not only do these microorganisms produce lipases, but they produce lipases that are, to the best of our knowledge, the only known lipases that naturally exhibit their maximal activity within the most desirable temperature and pH ranges for detergent applications. Maximal activities in such pH and temperature ranges are surprising, since the pH and temperature of these microorganisms' natural environments range from a pH of about 4 to about 7 and a temperature of about 0° to about 35° C, far lower than the temperature and pH ranges for detergent applications.

Since other lipases do not exhibit their maximal activities within the most desirable conditions for detergents, other lipases either must be used at a higher concentration than the lipases of this invention to achieve the same cleaning effectiveness, or they must be specially formulated in order to provide the required temperature and pH stability at which they are most active. If the lipases of this invention are used at a lower concentration, allergic reactions commonly induced by proteins may be reduced or even eliminated.

Because the lipases of this invention naturally exhibit their maximal activities within the most desirable temperature and pH range for detergent applications, it may not be necessary to formulate the lipases in such a way as to alter their maximal activities so that they are within the desired temperature and pH ranges. As a result, there may be more latitude in formulating the detergent in other ways so as to achieve other desirable properties, and a more effective detergent can therefore be made.

The lipases of this invention may be stable in the presence of detergent proteases, which permits the formulation of a multi-enzyme-based detergent that can include, inter alia, both lipases and proteases, and therefore that can effectively attack both fatty/oily stains and protein stains.

Figure 1 is a graph comparing the percent normalized enzyme activity of a lipase preparation of the present invention with the normalized enzyme activity of a commercially available detergent lipase; the drawing is more fully described in Example 1.

## The Microorganisms

The unique lipases of this invention are derived from microorganisms capable of producing lipases and of surviving and propagating in an environment containing high levels of halogenated organic compounds. Such microorganisms are prokaryotes or eukaryotes such as bacteria, fungi, actinomycetes, and archebacterium. Bacteria, particularly gram negative, rod shaped, non-fermentative, aerobic, motile bacteria, and more particularly members of the genus Pseudomonas, are the preferred microorganisms of this invention as they are more likely to produce the desired enzymes. Environments where such organisms can be found are, for example, those where there is a history of exposure to halogenated organic compounds. Examples of such halogenated organic compounds include 1,1-bis(4-chlorophenyl)-2,2,2-trichloroethane (DDT), 2,6-dichloro toluene, 3,4-dichloro benzoate, chlorinated benzenes, polychlorinated biphenyls (PCB), chlorinated phenols, and the like.

The particular halogenated organic compounds that are present in the environment of these microorganisms may vary, as may their concentrations, but all of the lipases useful in this invention are produced from genetically active material that is substantially identical to the lipase-encoding genetic material found in microorganisms that are capable of surviving and propagating (under laboratory test conditions) in the presence of 100 ppm of 3,4-dichlorobenzoate. This lipase-encoding genetically active material may exist in microorganisms found in highly halogenated environments, but it can also exist in microorganisms that are not found in such environments and that may or may not be capable of surviving and propagating in such environments. For example, a microorganism capable of surviving and propagating in such an environment could be transported (e.g., by wind or water) to a less inhospitable environment. Alternatively, the lipase-encoding genetic material in a microorganism that is capable of surviving and propagating in the highly halogenated environment may be transferred, using, for example, recombinant DNA technology, to another microorganism that is desired for use in commercial lipase production but which is incapable of surviving and propagating in an environment with high levels of halogenated organic compounds. (For the purposes

of this invention such microorganisms are genetic equivalents of the microorganisms that contain the lipase-encoding material.) Alternatively, the lipase-encoding genetic material can be synthesized in vitro and used to produce the lipase, possibly without even being placed within a living organism, or the lipase itself can be synthesized in vitro.

While microorganisms that are capable of producing the lipases of this invention exist in nature, in their natural state they have no apparent requirement to produce lipases due to a lack of fat or lipid substrates in the environment, and it is not known whether they produce any lipase at all in nature. If they do produce lipases in their natural state, the lipases would not be useful to man because they would be too difficult to isolate and purify. The microorganisms are useful in making lipases for detergents only when they have been isolated as substantially pure strains. Such pure strains can be obtained using procedures well known in the art by, for example, the use of techniques such as the soil dilution technique or the Warcup soil plating technique, in conjunction with enriching for these strains by employing selective techniques (e.g., ability to survive in and/or degrade specific halogenated organic compounds), and then through the repetitive transfer and subculture of the lipase-producing strains to ensure their biological purity. Those microorganisms that produce the desired lipases are then propagated in a suitable culture medium. For example, the cultures can be placed in a typical fermentation broth and held at a temperature within the range of 10°C to 45°C for from about 1 to about 3 days. The inoculation range can be 0.01% to 10% by volume based on total culture volume (v/v), but preferably is 2% to 5% v/v. The nutrient medium may include a variety of organic carbon sources and a source of available nitrogen.

In order to maximize production of the lipase during fermentation, a lipase production inducer may be added to the fermentation broth. The lipase production inducer is any suitable fat or oil, such as an acylglyceride. It is generally used in an amount of about 0.01% to about 0.05% v/v. Preferably, the microorganism should produce the enzyme extracellularly as that simplifies the purification process, but intracellular production is also possible.

The preferred extracellular lipases can be harvested by first removing the producing cells from the spent production medium. The removal of cells and cellular debris can be achieved by centrifugation, microfiltration, or other appropriate techniques. The lipases can then be purified further by ultrafiltration of the cell-free, lipase-containing production medium. The lipase also can be purified by a variety of methods including ammonium sulfate precipitation, affinity column purification, isoelectric focusing, freeze drying, acetone or ethanol precipitation, etc. (For further details with regard to standard techniques for fermentation and removal of cell debris from the lipase enzyme, see Atkinson, Handbook of Fermentation Technology, pp. 999-1015.)

## The Lipases

The lipases of this invention are substantially pure enzymatic proteins. Substantially pure lipases are required for use in detergents since impurities may reduce enzymatic activity. The lipases are capable of wholly or partially hydrolyzing ester linkages, such as those of triglycerides, the principal constituents of fats and oils, to glycerol and fatty acids:

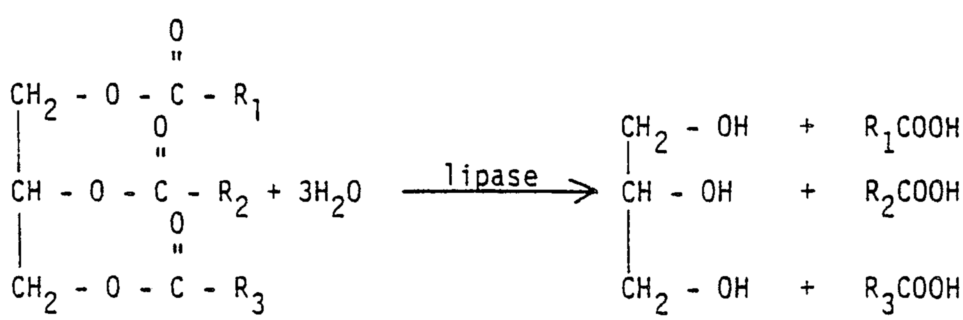


triglyceride glycerol fatty acids

where $R_1$, $R_2$, and $R_3$ are the same or different organic radicals. However, the lipases have not been found to transesterify olive oil and are not expected to transesterify other triglycerides or other esters, nor have they been found to degrade polyester fabrics, which are aromatic polymers.

The lipases of the present invention have their maximal lipolytic activity (measured against olive oil) at a temperature between about 20° C and about 85° C, preferably between 40° C and 60° C, at a pH between about 8.0 to about 11, preferably between about 9.0 and about 10.5. These lipases have molecular weights of about 10,000 to about 40,000, and preferably about 15,000 to about 35,000.

## The Lipase Genes

The invention also relates to the lipase encoding genes of the above-identified microorganisms, preferably of said bacteria of the present invention. Preferably such genes encode lipases having the biological properties and activities as outlined hereinabove.

The invention also relates to lipase-encoding genes having a DNA sequence which hybridizes to said genes and which encodes a lipase having the biological properties and activities as outlined hereinabove. Preferably these hybridizing DNA sequences encode a lipase having its maximal lipolytic activity between about 20° C and about 85° C at a pH between about 8.0 and about 11. In this context the term "hybridization" refers to hybridization conditions under which the $T_m$ value preferably is between $T_m$-20 to $T_m$-27. Most preferably the term "hybridization" refers to stringent hybridization conditions.

## The Lipase Composition

While the lipases themselves can be used as detergent components or as cleaning agents, it is preferable to first prepare a lipase composition and then use the lipase composition to formulate the detergent in order to stabilize and protect the lipase and possibly enhance its activity. A lipase composition can be made by mixing about 0.1% to about 5% by weight, preferably about 0.5% to about 1% by weight (based on lipase composition weight), of the active lipase with about 95% to about 99.9% of a suitable diluent (carrier). The diluent may be an inert substance or it may be selected to enhance the cleaning performance of the lipase and its stability on the shelf and/or in the wash cycle. If the lipase composition is to be a solid, diluents such as titanium dioxide, polyoxyethanolates, and waxes can be used. In order to obtain particles of the lipase composition that are approximately the same size as other particles in the detergent, generally about 0.5 to about 1.5 mm, the composition can be extruded and chopped into granules. Coatings such as mineral oil and starches, to maintain particle hydration and flowability, respectively, may also be used on the granules if desired. (For processing specifics see Novo Industri U.S. Patent 4,106,991, Gist-Brocades U.S. Patent 4,242,219, and Novo PCT Patent 8707292, which are herein incorporated by reference.) If a liquid or gel lipase composition is to be prepared, a diluent such as polyethylene glycol can be used. It is usually also desirable to include a preservative in a liquid or gel lipase composition to prevent degradation of the lipase.

## The Detergent

Detergents incorporating the lipases of this invention can be prepared using a variety of chemicals well known in the detergent formulating art. Since the presence of surfactants in a detergent can reduce the activity of an enzyme, detergent compositions that rely primarily or solely upon enzymes rather than surfactants to solubilize soil should contain less than about 30% by weight surfactant. A typical detergent, for example, may contain, in addition to the lipase composition of this invention, about 0.1% to about 40% by weight detergent active organic surfactant, and preferably about 1 to about 5% surfactant. Examples of detergent-active surfactants can be found in Schwartz, Perry and Berch, Surface-Active Agents and Detergents, Vol. I (1949) and Vol. 11 (1958) and in Schick, Nonionic Surfactants, Vol. I (1967). Anionic surfactants alone are preferably avoided as they may tend to inactivate lipases. (See European patent 0,097,810.) If used, anionic surfactants should be blended in after the addition of a nonionic surfactant or other stabilizer. In general, the weight ratio of the nonionic to the anionic surfactant can vary from 12:1 to 1:12, preferably from 8:1 to 1:8, and particularly preferably from 4:1 to 1:4. The amount of nonionic and anionic detergent-active surfactant together in the detergent composition can range from about 1% to about 30%, usually from 2% to about 20%, and preferably from 6% to about 16%, by weight. Surfactants of other types, such as cationic and zwitterionic surfactants, may also be included.

The detergent furthermore may include other usual detergent ingredients in the usual amounts. They may be built (contain STPP or similar components which serve to raise the pH of the wash solution and to

complex ions such as calcium and magnesium ions) or unbuilt, and may be of the zero-P type (i.e., not containing phosphorus-containing builders). The detergent may contain from about 1% to 45%, preferably from about 5% to 30%, (by weight) of one or more organic and/or inorganic builders. Typical examples of such builders are the alkali metal ortho-, pyro- and tripolyphosphates, alkali metal carbonates, either alone or in admixture with calcite, alkali metal citrates, alkali metal nitrilotriacetates, carboxymethyloxysuccinates, zeolites, polyacetylcarboxylates, and so on. A detergent can also contain about 4% to about 8% by weight alkali metal (e.g., sodium) silicate and about 0.5% to about 1% by weight carboxymethyl cellulose. Furthermore, a detergent may contain from about 1% to about 35% by weight of a bleaching agent or a bleaching system comprising a bleaching agent and an activator therefor. The detergent may include lather boosters, foam depressors, anti-corrosion agents, soil-suspending agents, sequestering agents, anti-soil redeposition agents, perfumes, dyes, stabilizing agents for the enzymes, brighteners, and so on.

In addition to the lipases of this invention, the detergent also preferably contains enzymes such as proteases, amylases, and/or cellulases. For example, in order to enable the detergent to solubilize protein stains, such as blood, it is desirable to include a protease in the detergent. An amylase, which solubilizes starches by converting them into simple sugars, may also be included in the detergent. As an example, a typical liquid detergent may contain:

(a) about 0.2% to about 1% by weight of the lipase composition of this invention;

(b) about 0.2% to about 1% by weight protease composition, which may contain about 0.1% to about 5% by weight (based on protease composition weight) active protease and the remainder diluent;

(c) about 0.2% to about 1% by weight amylase composition, which may contain about 0.1% to about 5% by weight (based on amylase composition weight) active amylase and the remainder diluent;

(d) about 10% to about 40% by weight surfactant;

(e) about 5% to about 15% by weight stabilizer, such as liquid propylene glycol or sorbitol;

(f) about 0% to about 15% by weight sequestering agent (a chelate such as ethylene diamine tetracetic acid or nitrilotriacetic acid); and

(g) about 0% to about 45% water.

A typical solid detergent may contain:

(a) about 0.1% to about 5%, and preferably about 0.2% to about 2%, by weight of the lipase composition of this invention;

(b) about 0.4% to about 0.8% by weight protease composition, which may contain about 0.1% to about 5% by weight (based on protease composition weight) active protease and the remainder diluent;

(c) about 0.4% to about 0.8% by weight amylase composition, which may contain about 0.1% to about 5% by weight (based on amylase composition weight) active amylase and the remainder diluent;

(d) about 1% to about 10%, and preferably about 2% to about 4%, by weight surfactant;

(e) about 0% to about 40% by weight builder (such as sodium tripolyphosphate); and

(f) about 15% to about 30% by weight bleach (such as sodium perborate);

Fat-like and oil-like soil and other soil can be removed from fabrics by washing the fabrics in an aqueous solution of a detergent according to this invention. Such detergents can be used at a temperature of about 5° C to about 80° C, preferably about 40° C to about 60° C, The pH of the washing solution may be about 7.0 to about 11, preferably about 9 to about 10.5, at concentrations of about 1 to about 20 grams per liter of washing solution.

Although the maximal activity of the lipases of this invention is at a temperature of about 40° C to about 60° C at a pH of about 9.0 to about 10.5, they can, of course, be used at other temperatures and pHs and in applications other than in detergents. For example, the lipases may be used in laundry prespotting and presoaking compositions, in hard surface cleaners, in oven cleaners, in degreasing metals and machine surfaces, and in cleaning lipid deposits from contact lenses and other optical devices. They may have industrial uses in dry cleaning, cleaning electrical components, chemical transformation reactions such as the hydrolytic breakdown or interconversion of triglycerides, and in chemical syntheses involving the breakdown of fatty materials. They also may be useful in environmental or process stream waste control. They may also have therapeutic uses in wound debridement and disinfection.

The following examples further illustrate this invention.

## EXAMPLE 1

Six different strains of Pseudomonas bacteria were collected at sites containing halogenated (primarily chlorinated) organic compounds at concentrations of at least about 150 ppm, and all six strains are capable

of surviving and propagating in an environment containing 100 ppm of 3,4-dichlorobenzoate. The temperature range at the sites was about 0° to about 35° C and the pH was about 4 to about 7, but unexpectedly, the lipases produced from the strains had their maximal activity at a temperature of 40 to 60° C and a pH of 9.0 to 10.5.

Samples of these strains were deposited with the American Type Culture Collection, 12301 Parklawn Drive, Rockville, MD 20852, prior to the filing date of this patent application, and are available therefrom upon the issuance of this application as a patent. Table 1 gives the American Type Culture Collection (ATCC) accession number, and some of the taxonomic information for each strain.

TABLE 1

| | ATCC ACCESSION NUMBERS | | | | | |
|---|---|---|---|---|---|---|
| | 53848 | 53849 | 53850 | 53851 | 53852 | 53853 |
| Morphology | Rod | Rod | Rod | Rod | Rod | Rod |
| Gram-Stain | Negative | Negative | Negative | Negative | Negative | Negative |
| Oxidase | + | + | + | + | + | + |
| Catalase | + | + | + | + | + | + |
| Motility | + | + | + | + | + | + |
| Pigment | - | None-Yellow | Green | Yellow | Yellow | Yellow |
| OX-FERM.[1] | | | | | | |
| Glucose | 0 | 0 | 0 | 0 | 0 | - |
| Sucrose | - | - | - | - | - | - |
| Lactose | - | - | - | - | - | - |
| UTILIZATION | | | | | | |
| Arabinose | - | - | +(-) | + | + | +(-) |
| Lactose | - | - | - | - | - | - |
| Fructose | + | - | + | + | + | - |
| Mannose | + | - | - | + | + | - |
| Tryptophan | - | - | - | - | +/- | - |
| Citrate | +/- | +/- | + | + | + | - |
| Adipate | - | - | + | - | - | - |
| Ethanol | + | - | + | - | + | - |
| Acetate | + | - | + | + | + | - |
| Sucrose | - | - | - | - | - | - |
| Mannitol | - | - | + | + | + | + |
| Growth (° C) | | | | | | |
| 25 | + | + | + | + | + | + |
| 35 | + | + | + | + | - | + |
| 41 | +/- | +/- | + | - | - | +/- |
| Gelatin Hydrolysis | - | + | - | + | - | + |

[1]Oxidation-Fermentation

These six bacterial strains are all characterized as saprophytic and all are capable of degrading hydrocarbons. ATCC No. 53850 was derived from ATCC No. 31944, which is described in U.S. Patent 4,477,570, issued October 16, 1984, in the name of Colaruotolo et al.

The techniques employed to isolate the six bacterial strains involved either the ability of the microorganisms to survive in the presence of high concentrations of a variety of halogenated organic compounds (up to 1000 ppm of selected haloorganic) or the ability to utilize selected halogenated organic compounds as a sole source of carbon and energy. The cultures, prior to production of lipase, were maintained on NBY medium (e.g., Nutrient broth (Difco) plus 0.2% yeast extract, solidified by the addition of 15 g/l agar) at a temperature of about 27° C to about 30° C.

To produce the lipases, cultures were transferred from the solidified NBY maintenance medium to fresh

NBY maintenance medium and were incubated at 27° C to 30° C. After the cells had multiplied sufficiently, they were transferred to a small volume of liquid production medium, which was either NBY, M9GOC, or M9GOCY. (M9GOC contains 0.1% glucose, 0.05% olive oil, and 5% casamino acids, plus 6 g/l $Na_2HPO_4$, 3 g/l $KH_2PO_4$, 0.5 g/l NaCl, 1 g/l $NH_4Cl_1$, 2 ml/l of a 1 M solution of $MgSO_4$, and 0.1 ml/l of a 1 M solution of $CaCl_2$. M9GOCY is M9GOC plus 0.02% yeast extract.) The cells were incubated at 27-30° C, then the cultures were transferred to a larger volume of the appropriate production medium and scaled up. For some of the six strains, a small amount of acylglyceride-containing lipase production inducer (e.g., olive oil) was added to accelerate and/or increase the production of the lipases. The microbial growth and the production of the lipases were monitored with respect to time. The fermentation was terminated when the lipase activity reached the desired level, which was monitored by olive oil assay. This assay measures the enzymatic hydrolysis activity of the lipase on the substrate olive oil.

Table 2 gives the percent normalized enzyme activity versus pH for the lipase preparations of the six strains against olive oil at 25° C, and Table 3 gives the percent normalized enzyme activity versus temperature for the lipase preparations from these six strains using olive oil as the substrate at a pH of 8.0. Activity refers to the ability of the lipase to hydrolyze triglycerides (such as those contained in olive oil) to their corresponding free fatty acids and glycerol. Percent activity was calculated for each lipase preparation by assigning a value of 100% to that point which required the greatest amount of alkali (NaOH) to neutralize the liberated fatty acid. The other data points for that lipase preparation were then calculated in reference to this highest value, to give the percent normalized activity values for each preparation.

Table 2

| Percent Normalized Enzyme Activity vs. pH | | | | | | | | |
|---|---|---|---|---|---|---|---|---|
| Lipase Prep. From ATCC # | Induced (I) Non-Induced (N) | 8.0 | 8.5 | 9.0 | 9.5 | 10.0 | 10.5 | 11.0 |
| 53849 | N | 15 | 44 | 56 | 100 | 93 | 78 | 54 |
| | I | 18 | 54 | 79 | 69 | 100 | 74 | 77 |
| 53850 | N | 19 | 31 | 71 | 100 | 60 | 50 | 54 |
| | I | 23 | 60 | 58 | 100 | 75 | 63 | 65 |
| 53848 | N | 19 | 53 | 66 | 100 | 98 | 62 | 19 |
| | I | 46 | 48 | 67 | 93 | 100 | 79 | 54 |
| 53851 | N | 45 | 53 | 61 | 86 | 100 | - | 43 |
| | I | 31 | 42 | 82 | 100 | 88 | 65 | 56 |
| 53853 | N | 29 | 46 | 75 | 100 | 66 | - | 44 |
| | I | - | 43 | 66 | 100 | 94 | 38 | 47 |
| 53852 | N | 28 | 49 | 93 | 100 | 51 | 39 | 45 |
| | I | 21 | 49 | 69 | 100 | 73 | 58 | 52 |

Table 3

| Percent Normalized Enzyme Activity vs. Temperature | | | | | |
|---|---|---|---|---|---|
| Lipase Prep. From ATCC # | Temperature (° C) | | | | |
| | 25 | 30 | 40 | 50 | 60 |
| 53849 | 8 | 37 | 100 | 95 | 57 |
| 53850 | 1 | 28 | 22 | 100 | 67 |
| 53852 | 10 | 15 | 30 | 78 | 100 |
| 53851 | 8 | 12 | 44 | 100 | 74 |
| 53848 | 10 | 47 | 83 | 100 | 53 |
| 53853 | 17 | 34 | 57 | 77 | 100 |

Tables 2 and 3 show that the lipases produced by the six strains had their maximal activity within the temperature range of 40° to 60° C at a pH of 9.0 to 10.5, both within the desirable range for modern enzyme-based detergents, i.e., temperatures up to 80° C at a pH of 8.5 to 11.

Table 4 compares the maximal pH and temperature ranges of three commercially available detergent lipases with the lipase preparations of this example.

TABLE 4

| Lipase | Maximal Range/Activity pH | Maximal Range/Activity Temp °C | Source |
|---|---|---|---|
| Lipase P "Amano"[1] | 6 | ~52 | Pseudomonas fluorescens |
| Lipase CES "Amano"[1] | 7 | ~52 | Pseudomonas sp. |
| Lipolase™ 30 T[2] | 8 | 25 | Humicola lanuginosa |
| Lipase of this Invention[2] | 9-10.5 | 40 | ATCC #53849 |
| Lipase of this Invention[2] | 9-10 | 45-55 | ATCC #53850 |
| Lipase of this Invention[2] | 9-10.5 | 40-60 | ATCC #53848 |
| Lipase of this Invention[2] | 9-10.5 | 45-60 | ATCC #53851 |
| Lipase of this Invention[2] | 9-10 | 60 | ATCC #53853 |
| Lipase of this Invention[2] | 9-10 | 45-60 | ATCC #53852 |

[1]Data from Amano International Enzyme Co. Inc. product literature.
[2]Standard olive oil assay at 25° C, pH 8.0, activity (%)

Referring to the drawing for an illustration typical of the lipase activity of these six strains, the normalized enzyme activity at 25° C against olive oil for the lipase preparation from ATCC #53849 was compared with that for commercially available LIPOLASE(TM) 30-T, from Novo Industri. The drawing shows that the lipase of this invention achieved its maximal activity at a pH between about 9 and about 10, within the normal detergent wash solution range, while LIPOLASE(TM) 30-T exhibited only about 40% activity within this pH range.

In the above-described experiments, olive oil, rather than a synthetic oil (e.g., tributyrin) was used because the major constituents of olive oil more closely resemble those of common oily and fat-like laundry stains, such as those caused by human sebum. (Sebum itself was not used in these tests because it is difficult to obtain consistent lots of sebum in the quantity needed for these tests.) Enzyme activity assay results obtained with olive oil as the substrate can be extrapolated to activity on human sebum stains with some degree of confidence, allowing a person skilled in the art to conclude that a lipase capable of hydrolyzing olive oil also is likely to be capable of hydrolyzing sebum as well. The results of olive oil experiments can also be extrapolated to other fats, oils, and lipids that are similar in composition to olive oil. In fact, experiments have shown that the six strains of this invention are active in hydrolyzing a variety of oils and fats. Table 5 gives the results of lipase activity tests using other oily or fatty substances in a media of nutrient agar plus 0.2% yeast extract; AC medium (Difco) and PG medium (5 g Peptone, 10 ml glycerol per liter). All the media contained Nile Blue Sulfate (Manual of Methods for General Bacteriology, P. Gerhardt, editor-in-chief, ASM, Washington, DC, 1981, pg. 438). The testing prodedure was based on the color change which occurs as the fats are hydrolyzed by the lipase.

Table 5

| LIPOLYTIC ACTIVITY AGAINST SELECTED FATS AND OILS | | | | | |
|---|---|---|---|---|---|
| Strain Tested (ATCC #) | Tallow* | Lard | Corn Oil | Peanut Oil | Egg Yolk Cholesterol |
| 53853 | + | + | + | + | - |
| 53849 | + | + | +/- | + | + |
| 53848 | + | + | + | + | - |
| 53851 | + | + | + | + | - |
| 53852 | + | + | + | + | + |
| 53850 | + | + | + | + | - |

*50% beef-50% pork
\+ = activity observed on all media tested
\- = no activity observed
+/- = activity observed on some of the media tested

The broad scope of lipolytic activity of these six strains against many different fats and lipids demonstrates the likely success of the lipases of this invention in removing a wide variety of real world lipid/fatty stains under actual laundry conditions.

## EXAMPLE 2

Swatches of fabric (cotton/polyester; 3 by 4 inches; Scientific Services, NJ) stained with synthetic sebum and dust were washed using the following conditions. Flasks containing 200 ml of water (150 ppm hardness; Ca:Mg molar ratio of 2:1) and 0.2M tris(hydroxymethyl) aminomethane buffer pH 9.5 were prewarmed to 40 °C. Freeze-dried supernatant prepared as in Example 1 or "Lipolase™ 30-T" was added. As necessary, the pH was readjusted to 9.5. Two stained fabric swatches were added to each flask and agitated at 150 rpm. After 30 minutes, the wash solution was decanted and the swatches were rinsed twice using 200 ml room temperature distilled water each time. The swatches were allowed to air dry. The reflectance of the fabric was measured at 460 nm using a Macbeth spectrophotometer (UV light excluded; daylight spectrum). Table 6 gives the results.

Table 6

| Additives | LU*/1 Wash Solution | Reflectance Values |
|---|---|---|
| None | - | 47.0 |
| Lipase Preparation From ATCC #53850 | 120<br>185<br>245<br>305 | 54.1<br>56.3<br>53.4<br>53.4 |
| "Lipolase™ 30-T" | 250<br>500 | 49.8<br>48.8 |

* LU = lipase units, defined as micromoles of fatty acid generated per minute as determined by the standard olive oil assay at room temperature and pHs of maximal activity.
[a] 40 minute wash, buffer at pH 9.
[b] 120 minute wash, buffer at pH 9.

Table 6 shows that the lipase of this invention performed better in this wash test than did the commercial lipase.

## Claims

1. A biologically pure culture of a bacterium having lipase-encoding genetic material, selected from the group consisting essentially of bacteria having ATCC accession numbers 53848, 53849, 53850, 53851, 53852, 53853, mutants thereof, genetic equivalents thereof, and mixtures thereof.

2. Genetically active material substantially identical to the lipase-encoding genetic material of a bacterium according to Claim 1.

3. A lipase-encoding gene of a bacterium according to claim 1.

4. A lipase-encoding gene having a DNA sequence which hybridizes to a gene according to claim 3 and encodes a lipase having its maximal lipolytic activity between about 20° C and about 85° C at a pH between about 8.0 and about 11.

5. A method of preparing, in substantially pure form, lipases which have their maximal lipolytic activity between about 20° C and about 85° C at a pH between about 8.0 and about 11 comprising

(a) selecting a lipase-producing microorganism containing genetically active material substantially identical to lipase-encoding genetic material in a prokaryotic or eukaryotic microorganism capable of surviving and propagating in a laboratory environment of 100 ppm of 3,4-dichlorobenzoate;

(b) propagating said lipase-producing microorganism in a suitable nutrient medium;

(c) inducing said lipase-producing microorganism to produce said lipases; and

(d) separating and isolating said lipases from said nutrient medium and/or cellular debris, thereby obtaining said lipases in substantially pure form.

6. The method according to Claim 5, wherein said lipase-producing microorganism is a bacterium.

7. The method according to Claim 6, wherein said bacterium is gram negative, rod-shaped, non-fermentative, aerobic, and motile.

8. The method according to Claim 7, wherein said bacterium is of the genus Pseudomonas.

9. The method according to Claim 8, wherein said bacterium is a strain selected from the group consisting essentially of bacteria having ATCC accession numbers 53848, 53849, 53850, 53851, 53852, 53853, mutants thereof, genetic equivalents thereof, and mixtures thereof.

10. The method according to Claim 5, wherein said microorganism is a fungus.

11. The method according to Claim 5, wherein said microorganism is an actinomycetes.

12. The method according to Claim 5, wherein said microorganism is an archebacterium.

13. The method according to any one of Claims 5 to 12, wherein said lipase-producing microorganism produces said lipase extracellularly.

14. The method according to any one of Claims 5 to 13 wherein about 0.01 to about 0.05% v/v of acylglyceride lipase production inducer is added to said media.

15. The method according to any one of Claims 5 to 14, wherein said maximal lipolytic activity is between about 4° C and about 60° C and between about pH 9.0 and about pH 10.5.

16. In a substantially pure form, lipases which have maximal lipolytic activity at a temperature between about 20° C and about 85° C at a pH between about 8.0 and about 11, said lipase being produced from genetically active material substantially identical to the lipase-encoding genetic material of a prokaryotic or eukaryotic microorganism capable of surviving and propagating in a laboratory environment of 100 ppm of 3,4-dichlorobenzoate.

17. The lipase according to Claim 16 which is obtainable according to the method of any one of Claims 6 to 15.

18. The lipase according to Claim 16 or 17 which has a molecular weight of about 10,000 to about 40,000.

19. The lipase according to any one of Claims 16 to 18, wherein said maximal lipolytic activity is between about 40° C to about 60° C at a pH between 9.0 and about 10.5.

20. A lipase composition comprising

(a) about 0.1 to about 5% by weight of a lipase according to any one of Claims 16 to 19; and

(b) about 95% to about 99.9% of a diluent.

21. Granules comprising a lipase composition according to Claim 20.

22. Granules according to Claim 21 having a particle size of about 0.5 mm to about 1.5 mm.

23. The lipase composition according to Claim 20 in the form of a solution or gel.

24. A detergent comprising
(a) a detergent active organic surfactant; and
(b) a small but soil-removing effective amount of a lipase composition according to Claim 20 or 23.

25. The detergent according to Claim 24 which also contains about 1% to about 35% by weight of a bleaching agent.

26. The detergent according to Claim 24 or 25 which also contains about 1% to about 45% by weight of a phosphate builder.

27. A detergent comprising
(a) a lipase composition according to Claim 20 or 23;
(b) enzyme selected from the group consisting of proteases, amylases, cellulases, and mixtures thereof; and
(c) about 0.1% to about 40% by weight surfactant.

28. A liquid detergent comprising
(a) about 0.2% to about 1% lipase composition according to Claim 20 or 23;
(b) about 0.2% to about 1% protease composition comprising about 0.1% to about 5% protease;
(c) about 0.2% to about 1% amylase composition comprising about 0.1% to about 5% amylase;
(d) about 10% to about 40% surfactant;
(e) about 5% to about 15% stabilizer;
(f) about 0% to about 15% sequestering agent; and
(g) about 0% to about 45% water.

29. A solid detergent comprising
(a) about 0.4% to about 0.8% lipase composition according to Claim 20 or 23;
(b) about 0.4% to about 0.8% protease composition comprising about 0.1% to about 5% protease;
(c) about 0.4% to about 0.8% amylase composition comprising about 0.1% to about 5% amylase;
(d) about 1% to about 10% non-ionic surfactant;
(e) about 0% to about 40% builder; and
(f) about 0% to about 30% bleaching agent.

30. A process for removing fat-like deposits from a substrate, comprising contacting said substrate with a solution containing a small but effective amount of a lipase according to any one of Claims 16 to 19.

31. A method of hydrolyzing an ester linkage of a non-polymeric non-aromatic compound comprising contacting said compound with a solution containing a small but effective amount of a lipase according to any one of Claims 16 to 19.

32. A method of removing fatty or oily compounds from a fabric where said compounds contain ester linkages comprising washing said fabric with a detergent according to any one of Claims 24 to 27.

Figure 1

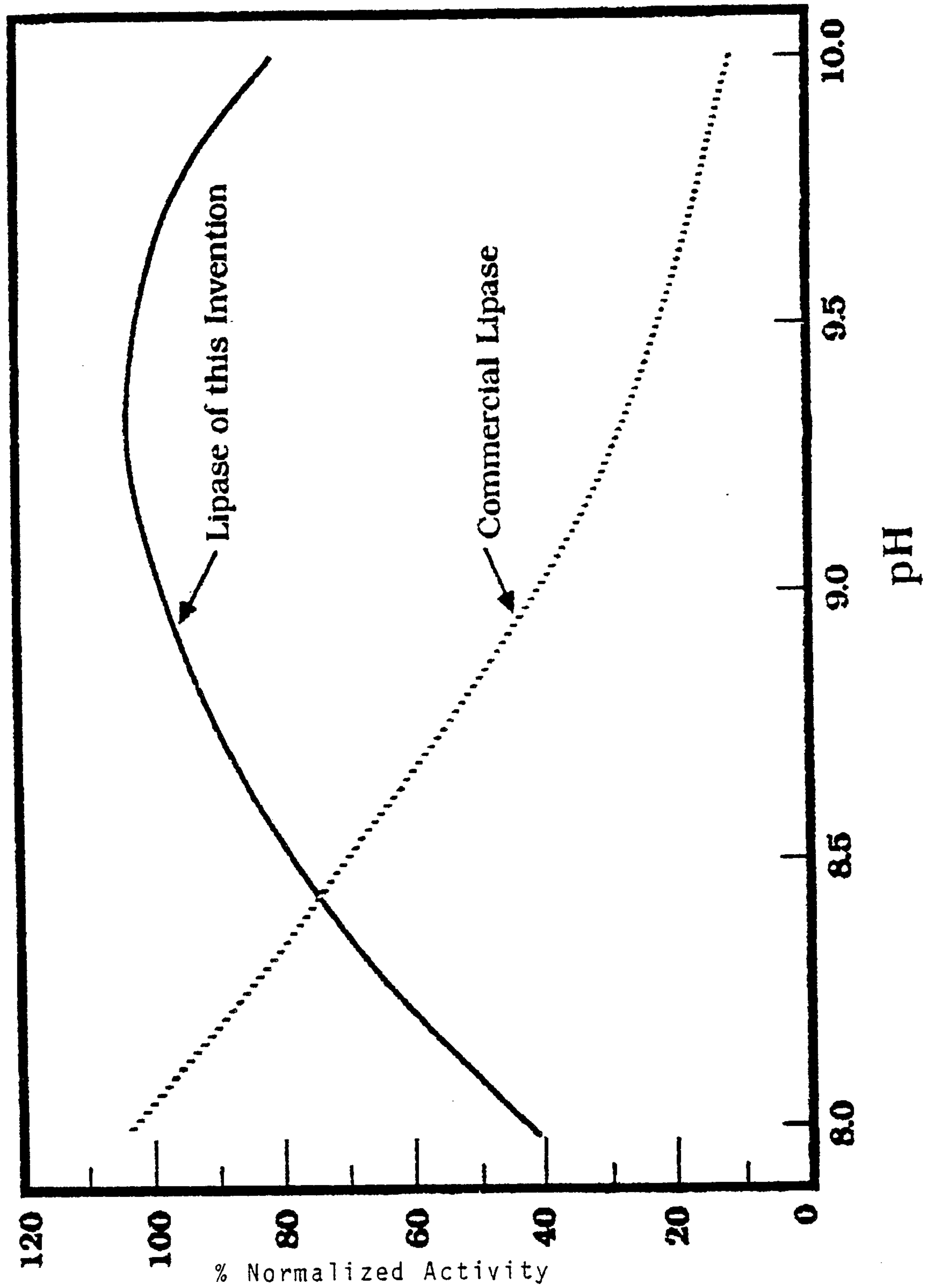
Lipase of this Invention
Commercial Lipase
pH
8.0
8.5
9.0
9.5
10.0
0
20
40
60
80
100
120
% Normalized Activity

European Patent Office

# EUROPEAN SEARCH REPORT

Application number EP 90103808.3

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| P,X | EP - A1 - 0 334 462 (GIST-BROCADES N.V.) * Claims 1,5,12-17 * -- | 1-8, 15-17, 19,24 | C 12 N 15/55<br>C 12 N 1/20<br>C 12 N 9/20<br>C 12 N 9/16<br>C 11 D 3/386<br>D 06 L 1/12<br>//(C 12 N 1/20,<br>C 12 R 1:38) |
| P,X | EP - A2 - 0 331 376 (AMANO PHARMACEUTICAL CO., LTD.) * Claims * -- | 1-8 | |
| A | PATENT ABSTRACTS OF JAPAN, unexamined applications, C field, vol. 12, no. 96, March 29, 1988 THE PATENT OFFICE JAPANESE GOVERNMENT Page 111 c 484 * Kokai-no. 62-228 279 (FUJI OIL CO LTD.) * -- | 2-8 | |
| A | EP - A2 - 0 243 338 (LABOFINA S.A.) * Claims * -- | 2-6 | **TECHNICAL FIELDS SEARCHED (Int Cl 5)** |
| P,A | EP - A1 - 0 330 641 (SYNFINA-OLEOFINA S.A.) * Claims * -- | 24,27 | C 12 N<br>C 11 D<br>D 06 L<br>D 06 M |
| D,A | PATENT ABSTRACTS OF JAPAN, unexamined applications, C field, vol. 12, no. 252, July 15, 1988 THE PATENT OFFICE JAPANESE GOVERNMENT Page 27 c 512 * Kokai-no. 63-39 579 (AMANO PHARMACEUT CO LTD.) -- | 24 | |
| D,A | JOURNAL OF APPLIED BIO-CHEMISTRY, vol. 2, 1980, New York | 24 | |

The present search report has been drawn up for all claims

| Place of search | Date of completion of the search | Examiner |
|---|---|---|
| VIENNA | 09-05-1990 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number

-2-

EP 90103808.3

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| | H.ANDREE et al. "Lipases as Detergent Components"<br>Pages 218-229<br>* Totality *<br>---- | | |
| | | | TECHNICAL FIELDS SEARCHED (Int Cl 5) |

The present search report has been drawn up for all claims

| Place of search | Date of completion of the search | Examiner |
|---|---|---|
| VIENNA | 09-05-1990 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document